# EUROPEAN PATENT APPLICATION

(11) **EP 2 316 386 A1**
(43) Date of publication of application: **04.05.2011**
(21) Application number: 10187454.3
(22) Date of filing: 13.10.2010
(51) Int. Cl.: A61F 2/40, A61F 2/00, A61F 2/02, A61F 2/30, A61F 2/46

(54) **Shoulder prosthesis component**

(30) Priority: 30.10.2009 US 609040
(71) Applicant: Depuy Products, Warsaw, IN 46581 (US)
(72) Inventor: Grant, Stuart R, Warsaw, IN 46580 (US)
(74) Representative: Belcher, Simon James

(57) **Abstract**

A humeral component of a shoulder prosthesis includes a head having a cavity. The head has a first bearing surface configured to mate with a glenoid bearing surface. The shoulder prosthesis also includes a stem having a recess defined therein, and the stem configured to be received by a humerus. The shoulder also includes a first connector having a proximal end and a distal end. The first connector also includes a threaded cavity and an outer wall. The outer wall engages the recess of the stem, and the threaded cavity opens at the proximal end of the first connector. A second connector having a threaded end sized and shaped to fit in the threaded cavity of the first connector is also included. The second connector also has a head connector end that is sized and shaped to fit within the cavity of the head. The outer wall of the first connector is sized and shaped to expand at the proximal end as the second connector is threaded into the threaded cavity.

## Description

The present invention relates to prosthetic devices particularly shoulder prostheses and, more particularly, to a shoulder prosthesis having a mechanism allowing adjustment of the position and orientation of the head.

The state of the prosthetic shoulder market has progressed such that a surgeon generally approaches shoulder replacement surgery in one of two strategic ways. One strategy is to perform the shoulder replacement surgery in accordance with a manufacturer's shoulder prosthesis or shoulder prosthesis product line. Particularly, a surgeon is provided with instrumentation and technique guidelines for the particular shoulder prosthesis or prosthesis line. The guidelines and/or instrumentation direct or dictate the angle of humeral head resection for the implant (prosthesis). This angle is in relation to the humeral intramedullary (IM) canal and is designed to match an optimum set of angles already present in the prosthetic design.

Another strategy is to perform the shoulder replacement surgery in accordance with a patient's anatomy. Particularly, the humeral head is resected according to angles perceived to be "anatomic" in the opinion of the surgeon, not according to angles already present in the prosthetic design. The prosthesis is designed such that the configuration of the prosthesis is adjustable intraoperatively. This allows the prosthesis to be adjusted so that it can match the bone preparation.

Even with respect to these two divergent approaches to surgical strategy, a common problem in shoulder surgery is matching the humeral resection angle to the predetermined angle designed into the prosthesis. This angle may describe the angle between a prosthetic collar and the diaphyseal section of the stem. In the case of a collarless stem, the angle may describe the difference between the long axis of the stem and the inferior surface of the prosthetic head. It is considered optimal for fixation and biomechanics if the resected angle and the angle of the prosthesis are identical, so as to allow intimate contact between the superior surface of resected bone and the inferior surface of the implant.

Moreover, the version angle at which the prosthesis is implanted will have a significant impact on the biomechanics of the prosthetic joint. Currently, most shoulder prosthesis systems on the market dictate the varus/valgus angle of the bone cut. This strategy does not allow the surgeon to easily alter biomechanics after the prosthesis has been trialed, much less implanted.

There are some known products currently marketed that attempt to resolve at least one of the above-noted issues. First, the system sold by Tornier under the trade mark Aequalis provides a modular junction within the metaphyseal region of the stem, which allows a small block between the stem and humeral head to be interchanged. This block is available in multiple angles, thus allowing the surgeon to select the block that best fits the boney anatomy as resected. This system, however, has two primary weaknesses. First, the use of modular blocks obviously forces the design to only allow angular adjustments in finite increments. Second, the need to adjust the angle through modular blocks forces the surgeon to remove the stem, change out a component, and reset the stem. This presents inconvenience, as well as risk for interfering with resected bone and compromising fixation.

The Anatomica product is sold by CenterPulse (and Zimmer) as a solution to the problems referred to above. This product provides a humeral head that is infinitely adjustable in varus/valgus and anterior/posterior angles relative to the stem portion of the prosthesis. This is accomplished through a spherical shaped protrusion on the superior surface of the stem that fits into a spherical recess in the humeral head. These mating surfaces allow the head to be articulated about the stem, thus allowing adjustable positioning of the head. The head can be locked in a position relative to the stem. This solution provides adjustment of the neck-shaft angle as well as being able to affect adjustment of the version through flexibility in the anterior/posterior angle. The locking means, however, is sub-optimal. Particularly, the locking mechanism requires the turning of a locking screw that has its head facing lateral and inferior, for which there is no access once the stem has been cemented. This eliminates the ability to adjust head position on the fly, and forces a total revision if articular surfaces ever need to be revised. Lastly, the protrusion on the humeral stem even when the humeral head is not in place limits the surgeon's access to the glenoid in preparation for a glenoid replacement.

In some cases, a spherical ball having a split on the top of the ball was discussed. The split spherical ball would engage a recess in the head. The idea is that the sphere allows the head to be rotated during surgery, but once the proper angle was chosen, the sphere can be expanded and lock into the recess. However, as the sphere expanded, the points of contact with the recesses move outward as the sphere expands. This results in the engagement between the sphere and the recess being unstable.

The present invention provides a shoulder prosthesis including a head having a cavity within it. The head further has a first bearing surface configured to mate with a second bearing surface of a glenoid, A stem having a recess defined therein is included and is configured to be received a humerus. The shoulder prosthesis also includes a first connector having a proximal end and a distal end. The first connector also includes a threaded cavity and an outer wall, the outer wall for engaging the recess of the stem. The threaded cavity opens at the proximal end of the first connector. The shoulder prosthesis further includes a second connector having a threaded end sized and shaped to fit in the threaded cavity of the first connector. The second connector also has a head connector end, sized and shaped to fit within the cavity of the head. The outer wall of the first connector is sized and shaped to expand at the proximal end as the second connector is threaded into the threaded cavity.

The invention also provides a shoulder prosthesis which includes a head having a cavity defined therein and a stem having a recess defined therein. The recess has a first open end that is defined in a proximal surface of the stem, a second closed end, and a sidewall extending between them. The shoulder prosthesis further includes a neck having a first coupling portion configured to mate in a friction fit manner with the cavity of the head, and a second coupling portion configured to be received in the recess of the stem. The neck includes a locking portion sized and shaped to lock the second coupling portion to the neck. The second coupling portion has a proximal end and a distal end and the locking portion engages the second coupling portion at the proximal end. The proximal end of the second coupling portion of the neck is configured to expand to thereby couple the neck to the stem when the second portion of the locking element is received within the proximal end of the second coupling portion of the neck.

Optionally, The shoulder prosthesis of claim 8, in which: the head further has a first bearing surface configured to mate with a second bearing surface of a glenoid, and the stem is configured to be received in an intramedullary canal of a humerus.

Optionally, the proximal end of the second coupling portion of the neck further has a plurality of coupler segments that are spaced apart from each other.

Optionally, the plurality of coupler segments includes three coupler segments, and each of the three coupler segments is separated from another of the three coupler segments by an axial slit.

Optionally, the second coupling portion includes a threaded cavity, sized and shaped to receive a threaded end the locking portion.

Optionally, the threaded cavity is tapered and the threaded end of the locking portion is cylindrical, such that as the threaded end of the locking portion is threaded into the tapered threaded cavity, the proximal end of the cavity expands.

Optionally, the neck is modular and the locking portion is separate from the second coupling portion.

The invention also provides a method of assembling a shoulder prosthesis for use in shoulder arthroplasty is provided. The method includes using a head having a cavity defined therein and a stem having a recess defined therein. A first connector having a proximal end and a distal end is also used. The first connector also includes a threaded cavity and an outer wall, the outer wall for engaging the recess of the stem. The threaded cavity opens at the proximal end of the first connector. The method also includes using a second connector having a threaded end sized and shaped to fit in the threaded cavity of the first connector. The second connector also has a head connector end, sized and shaped to fit within the cavity of the head. The method further includes inserting the first connector into the recess of the stem and adjusting the first connector to the desired angle. The second connector is then fully threaded into the first connector, causing the proximal end of the first connector to expand. This results in locking the first connector to the stem.

The shoulder prosthesis components provided by the invention allow adjustment of the angular position of the humeral head, especially through a wide range of angles.

The shoulder prosthesis components provided by the invention allow adjustment of the angular orientation of the humeral head during surgery.

The shoulder prosthesis components provided by the invention provide stability during the life of the device in service.

The shoulder prosthesis components provided by the invention allow parts thereof to be locked together once they are in the appropriate positions.

Embodiments of the invention are described below by way of example with reference to the accompanying drawings, in which:
FIG. 1 is an exploded perspective view of a shoulder prosthesis;
FIG. 2 is a perspective view of a neck of the shoulder prosthesis of FIG. 1;
FIG. 3 is a perspective view of a first connector of the prosthesis of FIG. 1;
FIG. 4 is bottom view of the first connector of FIG. 3;
FIG. 5 is a perspective view of a second connector of the prosthesis of FIG. 1;
FIG. 6 is a side sectional view of the stem and neck of FIG. 1;
FIG. 7 is a side sectional view of the stem and neck of FIG. 1 with the stem in a different orientation;
FIG. 8 is a side sectional view of the stem and neck of FIG. 1 with the neck in yet another orientation;
FIG. 9 is a side sectional view of the head, neck, and stem of FIG. 1 coupled together; and
FIG. 10 is flow chart illustrating a method of assembling a shoulder prosthesis.

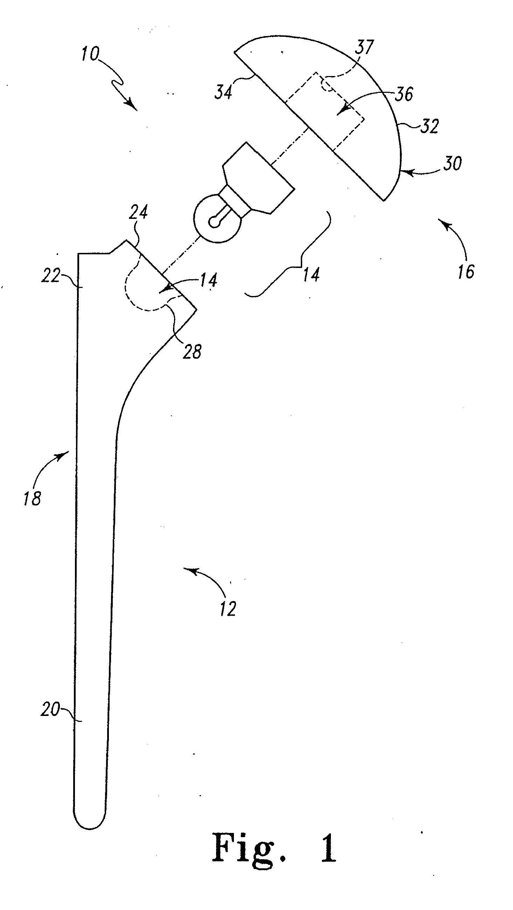

Referring to the drawings, FIG. 1 shows a shoulder prosthesis, generally designated 10 which includes a humeral component or stem 12, a neck 14, and a head 16. The head 16 is adapted to be coupled to the neck 14.

As shown in FIG. 1, the stem 12 includes a body 18 having a distal or stem portion 20 and a proximal or neck portion 22. The stem 12 may or may not have fins, collars, suture holes or the like. The proximal portion 22 has a preferably substantially flat or planar surface 24 having a recess 26 extending into the proximal portion 22. In one embodiment, an inner wall 28 defines the recess 26. The recess 26 may have a tapered inner wall 28. In other embodiments, the recess 26 may be cylindrical in shape.

The head 16 is characterized by a body 30 formed as a general partial spheroid. Particularly, the body 30 is shaped to conform to a glenoid. The body 30 has an articulation surface 32 conforming to the general partial spheroid and a bottom surface 34. It should be appreciated that the head 16 represents any size shoulder prosthesis head. The subject invention allows the use of various sized heads with the other components of the present shoulder prosthesis 10. While a head of only one size is ultimately used for the shoulder prosthesis 10 when implanted into the patient, the components of the present shoulder prosthesis 10 allow various sized heads to be trialed and/or used when the stem 12 is implanted into the humerus (i.e. during and/or after the time at which the stem 12 is final stage implanted in the humerus). The various heads may be variously proportioned and/or sized.

The head 16 further includes a recess, cavity or the like 36 defined by an inner surface 37 within the body 30 that is open on the underside or bottom surface 34. The inner surface (wall) 37 and thus the cavity 36 is tapered and will be described more fully below.

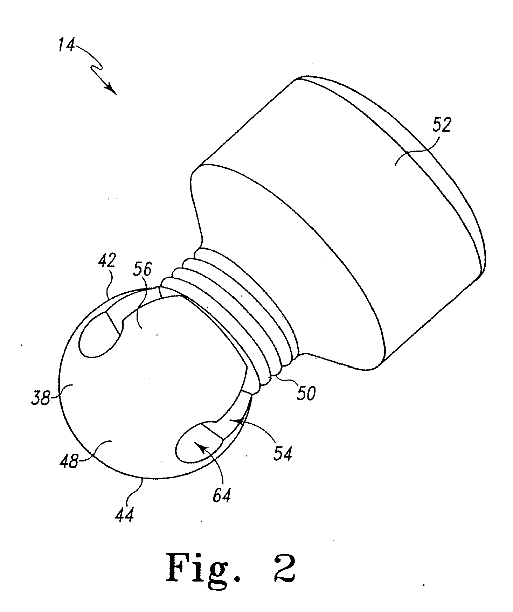

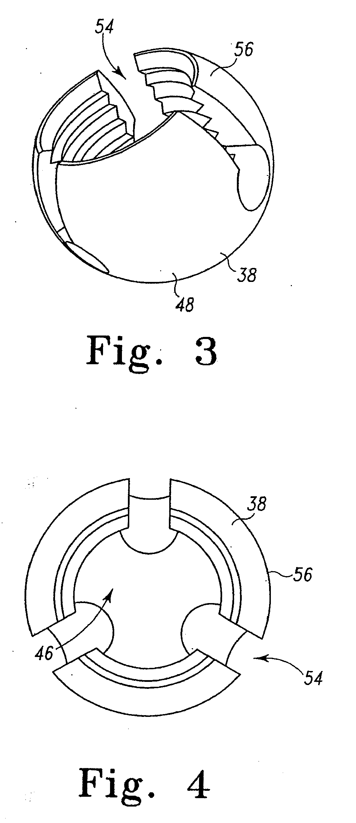

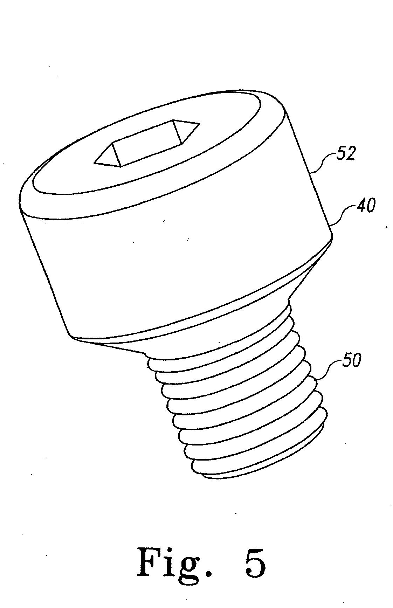

As shown in FIGS. 2 to 5, the neck 14 includes a first connector 38 and a second connector 40. The first connector 38 includes a proximal end 42 and a distal end 44. In other embodiments, the neck 14 may be a single piece, but with two connectors that are moveable relative one another. In some embodiments, the two connectors 38, 40 may be preassembled to be together. The first connector 38 includes a threaded cavity 46 and an outer wall 48. The outer wall 48 engages the recess 26 of the stem 12.

The second connector 40 includes a threaded end 50 and a head connector end 52. The head connector end 52 couples the second connector to the head 16. The head connector end 52 is in the shape of a male taper such that when the head connector end 52 is inserted into the cavity 36 of the head 16, the head connector end 52 is taper locked into the cavity 36. In other embodiments, the head connector end 52 may not be tapered and the head connector end 52 may be locked into the cavity 36 of the head 16 using other known methods.

The threaded end 50 of the second connector 40 is sized and shaped to thread into the threaded cavity 46 of the first connector 38. The threaded cavity 46 of the first connector 38 has a diameter that is slightly less than the diameter of the threaded end 50 of the second connector 40. As the threaded end 50 is threaded into the threaded cavity 46, the threaded end 50 forces the proximal end 42 of the first connector 38 to expand.

In the illustrated embodiment, the first connector 38 includes a plurality of slits 54 at the proximal end 42. The slits 54 are defined by a plurality of coupler segments 56. In this embodiment, there are three slits 54 and three coupler segments 56. In other embodiments, there may be other numbers of slits 54 and coupler segments 56. As the threaded end 50 of the second connector 40 is threaded into the threaded cavity 46, the slits 54 allow the coupler segments 56 to separate and expand.

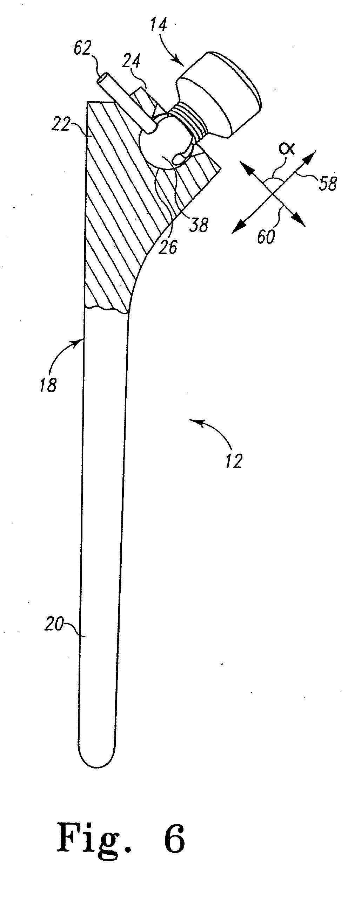

FIG. 6 shows the neck 14 after it is introduced into the recess 26 of the stem 12. Particularly, the first connector 38 (in the shape of a spheroid) is inserted into the recess 26.

The neck defines a longitudinal axis 58 and the planar surface 24 of the stem 12 defines a plane 60. After the neck 14 is inserted, the longitudinal axis 58 of the neck 14 and the plane 60 defined create an angle α. In FIG. 6, the angle α is 90°, indicating that there is not an offset between the neck 14 and the stem 12.

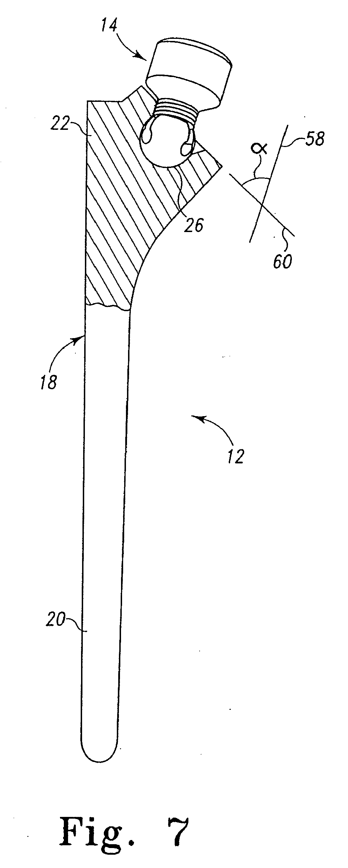

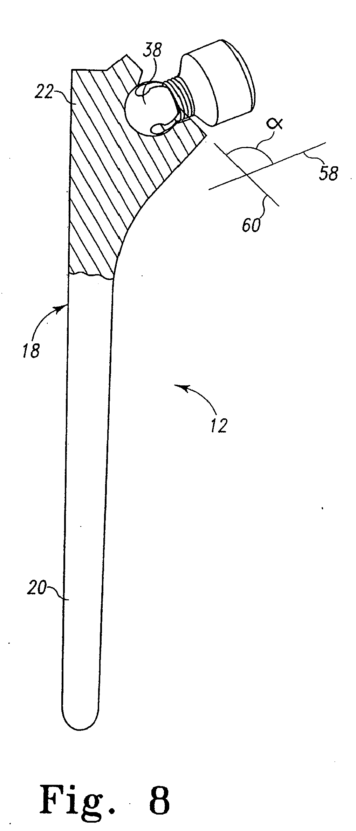

FIG. 7 depicts an offset of the stem 12 relative to the neck 14. In this embodiment, angle α is less than 90°. In other words, the neck 14 is angled to the left. FIG. 8 also depicts an offset of the stem 12 relative to the neck 14 since the angle α is more than 90° (i.e. the neck 14 is angled to the right). The stem 12 is infinitely adjustable along the two orthographic axes to set the spatial orientation of the stem 12 relative to the neck 14 and thus the head 16. This may be accomplished either before or after the stem 12 is set in the humerus of the patient.

It should be appreciated that FIGS. 6 to 8 only depict angular orientation relative to one axis of rotation. The other axis of rotation is orthographic to the one depicted and, while not shown, exhibits the same angular displacement in the respective spatial orientations.

Once the appropriate angular orientation of the stem 12 is determined, a pin 62 is inserted into a pin-receiving aperture 64 (FIG. 2) in the stem 12. The pin engages an aperture 64 formed in the slits 54 of the first connector 38 and effectively keeps the stem 12 and the first connector 38 in the same position relative to one another. The second connector 40 is then threaded into the first connector 38, with the threaded end 50 forcing the coupler segments 56 to push outward and engage the inner wall 28 of the recess 26 of the stem 12. Such radial expansion fixes the orientation of the stem 12 relative to the neck 14.

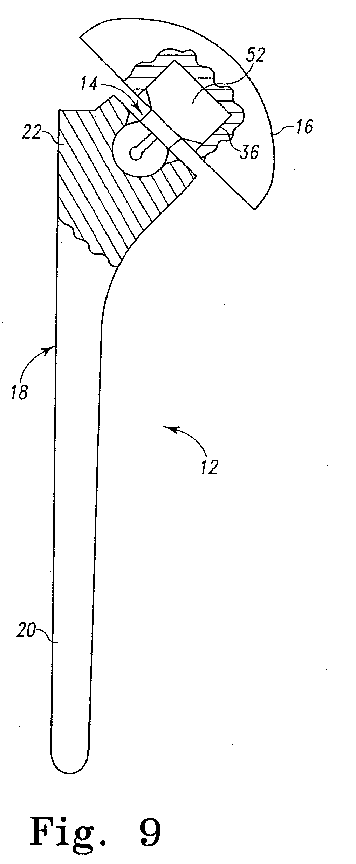

As depicted in FIG. 9, the neck 14 is releasably affixed to the head 16 by means of the head connector end 52. Once the angle α (as shown in FIGS. 6 to 8) is set, the head 16 can be coupled to the neck 14. The user couples the tapered head connector end 52 of the second connector 40 with the tapered cavity 36 of the head. Once the angle α is set and the surgeon is ready to attach the head, the surgeon can try on different size heads and/or change heads and spatially oriented while the stem 12 is implanted in the humerus and the angle α is set. Because the angle α is set, the user can try different sized heads while keeping the offset accurate.

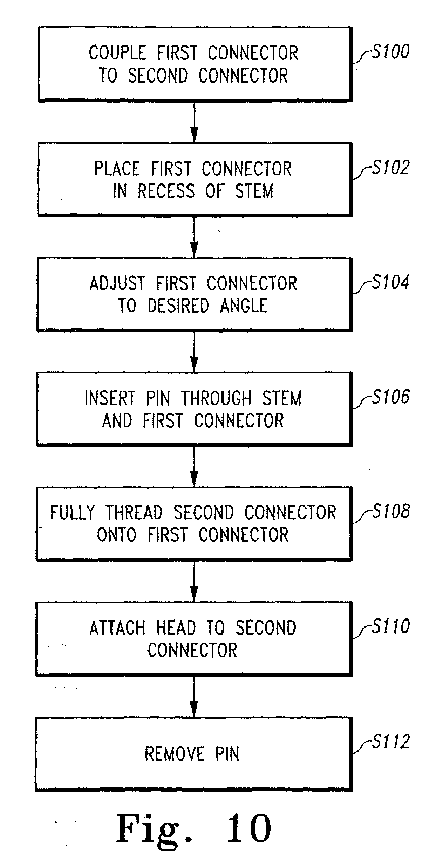

FIG. 10 shows steps in a method of assembling a shoulder joint prosthesis. At step s100, the first connector 38 is coupled to the second connector 40. The threaded end 50 of the second connector 40 is loosely threaded into the first connector 38. In some embodiments, the first connector 38 and second connector 40 may be preassembled. In other embodiments, they may be a single component that has two parts that are moveable relative to one another. The first connector 38 is then placed into the recess 26 of the stem 12 at step s102. The first connector 38 is then adjusted to the desired angle relative to the stem 12 at step s104. In some embodiments, once the desired angle is achieved, the pin 62 is placed through the pin aperture 64 in the stem and through first connector 38 at step s106. The second connector 40 is then fully threaded on to the first connector 38, causing the coupler segments 56 to expand and engage the wall 28 of the stem 12 (step s108). The expanding coupler segments 56 cause the neck 14 to be locked into the stem at the desired angle. The head 16 is then attached to the head connector end 52 of the second connector 40 at step s110. Although this embodiment illustrates a taper lock connection between the head 16 and the second connector 40, other known types of connections may be used. If the pin 62 is used, the pin 62 may be removed at step s112.

The shoulder prosthesis 10 may be made of any known biocompatible material. For example, the shoulder prosthesis 10 may be made of titanium, cobalt chrome and/or stainless steel. Other known biocompatible or medical grade implant materials may also be used.

The invention is applicable to shoulder prosthesis components which are intended for implantation in a surgical procedure. The invention is applicable to shoulder prosthesis components which are intended for use during a surgical procedure as trial components.

Although the drawings show the use of a long humeral stem which might extend into the intramedullary canal, it should be understood that this component of the prosthesis need not have a distal portion which extends into the intramedullary canal. The component is required simply to extend into the humerus.

## Claims

1. A shoulder prosthesis component, comprising:
a head having a cavity defined therein, the head further having a first bearing surface configured to mate with a glenoid bearing surface,
a stem having a recess defined therein, the stem being configured to be received in a humerus;
a first connector having a proximal end and a distal end, the first connector also includes a threaded cavity and an outer wall, the outer wall for engaging the recess of the stem, the threaded cavity opens at the proximal end of the first connector; and
a second connector having a threaded end sized and shaped to fit in the threaded cavity of the first connector, the second connector also having a head connector end, sized and shaped to fit within the cavity of the head;
in which the outer wall of the first connector is sized and shaped to expand at the proximal end as the second connector is threaded into the threaded cavity.

2. The shoulder prosthesis of claim 1, in which the outer wall of the first connector defines at least one slit that begin at the proximal end and extend toward the distal end.

3. The shoulder prosthesis of claim 2, in which the at least one slit is sized and shaped to allow the proximal end of the first connector to expand as the threaded end of the second connector is threaded into the first connector.

4. The shoulder prosthesis of claim 2, in which the stem includes a pin aperture for receiving a pin, and the at least one slit is sized and shaped to align with the pin aperture and to receive the pin such that the pin locks the first connector into place in the stem so that the first connector does not rotate as the second connector is threaded into the threaded cavity.

5. The shoulder prosthesis of claim 1, in which the recess defined in the stem is a tapered recess.

6. The shoulder prosthesis of claim 1, in which the recess defined in the stem is a cylindrical recess.

7. The shoulder prosthesis of claim 1 in which the thread connecting end and the cavity in the head connect by means of a taper lock.

8. A method of assembling a shoulder prosthesis for use in shoulder arthroplasty, the method comprising:
using a head having a cavity defined therein, a stem having a recess defined therein, a first connector having a proximal end and a distal end, the first connector also includes a threaded cavity and an outer wall, the outer wall for engaging the recess of the stem, the threaded cavity opens at the proximal end of the first connector, and a second connector having a threaded end sized and shaped to fit in the threaded cavity of the first connector, the second connector also having a head connector end, sized and shaped to fit within the cavity of the head;
inserting the first connector into the recess of the stem;
adjusts the first connector to the desired angle; and
fully threading the second connector into the first connector, causing the proximal end of the first connector to expand, locking the first connector to the stem.

9. The method of claim 8, which includes, after adjusting the first connector to the desired angle, inserting a pin through the stem and first connector, locking the first connector at the desired angle relative to the stem.

10. The method of claim 9, which includes, after fully threading the second connector into the first connector, removing the pin from the first connector and the stem.

11. The method of claim 8, which includes, after fully threading the second connector into the first connector, coupling the head on to the second connector.

12. The method of claim 11, in which the coupling comprises locking the head on to the second connector by means of a taper lock.

13. The method of claim 10, in which the proximal end of the first connector includes a plurality of slits defined by a plurality of coupler segments and when the second connector is threaded into the first connector, the plurality of coupler segments expand outwardly to engage the walls of the recess.
